**Europäisches Patentamt**

**European Patent Office**

**Office européen des brevets**

(11) Veröffentlichungsnummer: **0 308 790**

**A2**

# (12) EUROPÄISCHE PATENTANMELDUNG

(21) Anmeldenummer: **88114988.4**

(22) Anmeldetag: **14.09.88**

(51) Int. Cl.⁴: **C07D 487/22** , //(C07D487/22, 257:00,209:00,209:00,209:00, 209:00)

(30) Priorität: **21.09.87 DE 3731689**

(43) Veröffentlichungstag der Anmeldung: **29.03.89 Patentblatt 89/13**

(84) Benannte Vertragsstaaten: **AT BE DE FR GB NL**

(71) Anmelder: **Degussa Aktiengesellschaft Weissfrauenstrasse 9 D-6000 Frankfurt am Main 1(DE)**

(72) Erfinder: **Buchler, Johann, Prof. Dr. Richard-Wagner-Weg 3 D-6100 Darmstadt(DE)**
Erfinder: **Kruppa, Steffen Jahnstrasse 57 D-6078 Neu-Isenburg(DE)**
Erfinder: **Schmidt, Manfred, Dr. Unter-Haitzergasse 19 D-6460 Gelnhausen(DE)**
Erfinder: **Prescher, Günter, Dr. 10 Sherwood Drive Larchmont, N.Y. 10538(US)**

(54) **Rhenium-oxo-porphyrin-komplexe.**

(57) Es werden Rhenium-oxo-Komplexe mit Liganden

- Octaethylporphyrin oder
- 5,10,15,20-Tetraphenylporphyrinen oder
- 5,10,15,20-Tetra(4-pyridyl)-porphyrinen als Liganden,

welche gegebenenfalls am Zentralatom ein Anion tragen sowie Verfahren zur Herstellung der Komplexe vorgestellt.

EP 0 308 790 A2

## Rhenium-oxo-porphyrin-Komplexe

Die Erfindung betrifft neue Übergangsmetall-Komplexverbindungen mit

- Octaethylporphyrin oder
- 5,10,15,20-Tetraphenylporphyrinen oder
- 5,10,15,20-Tetra(4-pyridyl)-porphyrinen als Liganden,

welche gegebenenfalls am Zentralatom ein Anion tragen und welche sich als Katalysator zur Epoxidation von Olefinen mit Wasserstoffperoxid eignen.

Olefinoxide (Oxirane) sind Verbindungen von beträchtlicher industrieller Bedeutung. Sie finden Verwendung auf dem Gebiet der Lacke, zur Herstellung von Polyethern, Polyurethanen, Epoxidharzen, Detergentien, Glykolen und einer Vielzahl von organischen Zwischenprodukten (siehe US-PS 2 412 136 sowie DE-AS 11 39 477).

Zur Epoxidation von Olefinen sind schon verschiedene Verfahren bekannt. So lassen sich Oxirane nach der Chlorhydrinmethode durch Umsetzung von Olefinen mit Chlor oder Natriumhypochlorit in alkalischem Medium und nachfolgender Behandlung mit Basen herstellen.

Ein weiterer Prozeß beruht auf der Umsetzung von Olefinen mit organischen Hydroperoxiden in Gegenwart eines Katalysators (siehe DE-AS 14 68 012).

Ein weiteres Verfahren beruht auf der Verwendung von organischen Persäuren, die man durch Luftoxidation der entsprechenden Aldehyde oder aus Carbonsäuren mit Wasserstoffperoxid erhält (siehe BE-PS 535 068).

Damit verbundene Nachteile lassen sich durch Verwendung von Wasserstoffperoxid als Epoxidationsmittel beheben, da hierbei nach der Theorie neben dem Epoxidationsprodukt nur Wasser anfallen sollte. Da die Reaktivität des Wasserstoffperoxids gegenüber Olefinen schwach ist, werden Epoxidationen mit diesem Reagens unter Verwendung von Katalysatoren durchgeführt. In diesem Zusammenhang wird beispielsweise hingewiesen auf GB-PS 837,464, bei welchem die in J.A.C.S., Band 59, Seiten 2342 bis 2344 (1937) beschriebenen verschiedenen Metallkatalysatoren verwendet werden, auf US-PS 2 786 854, wonach Wolframsäure eingesetzt wird, auf US-PS 2 833 787, wonach saure Salze von Metallen aus der Gruppe VI des Periodensystems der Elemente, z.B. von Wolfram und Molybdän, angewandt werden, auf BE-PS 860 776, wonach Wolfram- und Molybdän-haltige Verbindungen verwendet werden, auf US-PS 3 993 673, wonach Arsen-haltige Katalysatoren verwendet werden, auf US-PS 3 953 362, wonach ein Molybdän-haltiger Katalysator angewandt wird, auf US-PS 4 026 908, wonach Quecksilberderivate plus eine Molybdän-, Wolfram-, Vanadin- oder Titanverbindung angewandt wird, auf US-PS 3 806 467, wonach organische und anorganische Zinnverbindungen plus organische oder anorganische Verbindungen, die Molybdän, Wolfram, Vanadin, Selen oder Bor enthalten, eingesetzt werden, auf Bull. Chem. Soc. Jap. 42, Seiten 1604 (1969), wonach Selendioxid angewandt wird und auf US-PS 3 778 451, wonach Molybadän-, Wolfram-, Vanadin-, Niob-, Tantal-, Uran- und Rheniumverbindungen eingesetzt werden.

Diese Stoffe sind zwar katalytisch aktiv, doch haben aus verschiedenen Gründen die damit grundsätzlich ausführbaren Verfahren keinen Eingang in die Technik gefunden. In Verbindung mit Wasserstoffperoxidlösungen wird durch sie entweder das Wasserstoffperoxid rasch zersetzt oder nur eine unbefriedigende Epoxidationsgeschwindigkeit erreicht. Verfahren mit diesen Katalysatoren sind auch insoweit problematisch, als neben dem gewünschten Epoxidationsprodukt häufig größere Mengen an Nebenprodukten, wie Diole und Ketone gebildet werden, deren Abtrennung erhebliche Schwierigkeiten bereiten kann.

Es sind auch schon Versuche unternommen worden, Verfahren zur katalytischen Epoxidation von Olefinen mit anderen Epoxidationsmitteln unter Verwendung von Metalporphyrinkomplexen als Katalysatoren durchzuführen. Als zur Umsetzung mit Epoxidationsmitteln wie Jodosobenzol, Alkalimetallhypochlorit sowie organische Hydroperoxide geeignete Metalkatalysatoren sind z. B. das Chloro-Eisen(III)-tetraphenylporphyrin(FeCl)(TPP), das Chloro-Mangan(III)-tetraphenylporphyrin(MnCl)(TPP) oder das Chloro-Chrom(III)-tetraphenylporphyrin (CrCl)(TPP) vorgeschlagen worden. Mangan(III)-tetraphenylporphyrin wurde auch bereits mit Wasserstoffperoxid als Oxidationsmittel eingesetzt (Renaud, J.-P.; Battioni, P.; Bartoli, J.F.; Mansuy, D., J. Chem. Soc., Chem. Commun. 1985, 888). Allerdings wirken diese Katalysatoren stark zersetzend auf $H_2O_2$, so daß die bezüglich Wasserstoffperoxid erreichbaren Selektivitäten nur sehr gering sind, es sei denn, es werden aufwendig substituierte Porphyrinliganden verwendet.

Auch Oxo-Metallporphyrinkomplexe, wie Oxo-chloro(5,10,15,20-tetraphenylporphyrinato)-molybdän (V) (O = Mo(TPP)Cl) sind in Verbindung mit organischen Hydroperoxiden schon vorgeschlagen worden. Ein Versuch, anstelle eines organischen Hydroperoxids Wasserstoffperoxid mit einem Katalysator der Zusammensetzung Methoxo-oxo(5,10,15,20-tetraphenylporphyrinato-molybdän(V) zur Epoxidierung des Olefins Cyclohexen zu verwenden, schlug jedoch fehl: Es konnte keine Epoxidation beobachtet werden (F. Varescon, These, L'Université Claude Bernard-Lyon I, 1982).

Gegenstand der Erfindung sind neue Rhenium-oxo-Komplexe mit

- Octaethylporphyrin oder
- 5,10,15,20-Tetraphenylporphyrinen oder
- 5,10,15,20-Tetra(4-pyridyl)-porphyrinen als Liganden,

welche gegebenenfalls am Zentralatom ein Anion, außer 0/2$^-$ (μ-oxo), $CH_3O^-$, $C_6H_5O^-$ oder $F^-$ bei den Grundkörpern, tragen, welche sich zur katalytischen Epoxidation von Olefinen mit Wasserstoffperoxid mit sehr hoher Selektivität eignen.

Die Komplexe können als Anion $Cl^-$, $Br^-$, $I^-$, $C_2H_5O^-$, $C_3H_7O^-$, $t-C_4H_9O^-$, $AcO^-$, $SCN^-$, $OCN^-$ und $ClO_4^-$ haben.

Bei den Grundkörpern können jeweils Wasserstoffatome oder freie Elektronenpaare an den Phenyl- bzw. Pyridylgruppen der Porphyrinliganden ein- oder mehrmals substituiert sein durch Halogen, Hydroxy, Carboxy, Cyano, Rhodano, Nitro, $C_1$-$C_6$-Alkyl, $C_1$-$C_6$-Alkoxy, Trihalogenmethyl, $C_1$-$C_6$-Alkansulfonyloxy, Aminocarbonyl, einen oder zwei $C_1$-$C_6$-Alkylreste enthaltendes Aminocarbonyl, $C_1$-$C_6$-Alkylcarbonyl, Amino, Di-$C_1$-$C_6$-Alkylamino, $C_1$-$C_6$-Alkanoylamino, $C_1$-$C_6$-Alkyl-$C_1$-$C_6$-alkanoylamino, $C_1$-$C_6$-Alkansulfonylamino, $C_1$-$C_6$-Alkyl-$C_1$-$C_6$-alkansulfonylamino, Aminosulfonyl, einen oder zwei $C_1$-$C_6$-Alkylreste enthaltendes Aminosulfonyl, $C_1$-$C_6$-Alkoxysulfonyl (-$SO_2$-O-$C_1$-$C_6$-Alkyl), Sulfo oder $C_1$-$C_6$-Alkansulfonyl und zweimal in o-Stellung durch die Methylendioxygruppe, und das Anion kann gegebenenfalls $Cl^-$, $Br^-$, $I^-$, $C_2H_5O^-$, $C_3H_7O^-$, $t-C_4H_9O^-$, $AcO^-$, $SCN^-$, $OCN^-$ und $ClO_4$ oder aber auch 0/2$^-$(μ-oxo), $CH_3O^-$, $C_6H_5O^-$ oder $F^-$ sein, wodurch ein etwa erforderlicher Ladungsausgleich bewirkt wird.

Durch die sterischen und elektronischen Effekte der genannten und anderer Substituenten am Phenyl- bzw. Pyridylrest des 5,10,15,20-Tetraphenylporphyrins bzw. 5,10,15,20-Tetra(4-pyridyl)-porphyrins kann man, angepaßt an das jeweilige Olefin, die katalytischen Eigenschaften steuern und optimieren.

Die erfindungsgemäßen Katalysatoren sind neue Stoffe. Davon ist eine Reihe nach bekannten Literaturmethoden in großer Reinheit zugänglich:
J. W. Buchler et al., Chem. Ber., 1973, 106, 2710; Liebigs Ann. Chem. 1971, 745, 135; Inorg. Nucl. Chem. Lett., 1972, 8, 1073; K. rohbock, Dissertation, RTWH Aachen, 1972; H. Stoppa, Dissertation RWTH Aachen, 1976. Die verschiedenen Porphyrinliganden werden, sofern sie nicht käuflich sind, nach Adler et al., J. Org. Chem.32, 476 (1976) bzw. Adler et al., J. Heterocyl. Chem. 5, 669 (1968) dargestellt und sofern erforderlich, von Chlorin (Porphyrin mit einem teilhydrierten Pyrrolglied) befreit (K.M. Smith et al., Tetrahedron Lett., 30, 2887 (1973))>

Oxorhenium(V)-Porphyrine erhält man aus $Re_2O_7$ in der Phenolschmelze in einem relativ langwierigen und umständlichen Verfahren (siebenwertiges Rhenium wird zu fünfwertigem reduziert). Bisher wurden nur zweikernige Porphyrinderivate des einwertigen Rheniums mit Kohlenmonoxid als weiteren Liganden hergestellt; diese haben z. B. die Zusammensetzung $Re_2(CO)_6(TTP)$ (M. Tsutsui et al., J. Am. Chem. Soc. 94, 7603 (1972), ibid. 98, 7878 (1976), ibid. 99, 620 (1977). Der hier vorgestellte Rheniumeinbau über Rheniumpentahalogenide, z. B. Rheniumpentachlorid, führt in glatter Reaktion ohne Änderung der Oxidationsstufe zu sehr guten Ausbeuten an Rheniumporphyrinen.

Ein weiterer wichtiger Gegenstand der Erfindung ist daher ein Verfahren zur herstellung von Rhenium-oxo-Komplexen allgemein, insbesondere aber der erfindungsgemäßen neuen Rhenium-Komplexe. Das Verfahren ist dadurch gekennzeichnet, daß man ein Mol Octaethylporphyrin, 5,10,15,20-Tetraphenylporphyrin oder 5,10,15,20-Tetra(4-pyridyl)-porphyrin mit mindestens einem Mol Rheniumpentahalogenid und 1 Mol Wasser in einem hochsiedenden, alle Reaktanden lösenden Solvens bei Temperaturen von 160 - 250, vorzugsweise 180 - 220° C, umsetzt, das Solvens vorzugsweise im Vakuum entfernt, den Rückstand nach Aufnehmen in einem organischen Lösungsmittel durch Säulenchromatographie fraktioniert, dann die in der Hauptfraktion vorliegenden, hinsichtlich der Zusammensetzung des Anions noch inhomogenen Komplexe entweder jeweils mit einer äquimolaren Menge einer gegebenenfalls in situ erzeugten Alkalimetallverbindung, welche das gewünschte Anion in Bindung mit dem Alkalimetall enthält, bei 0 - 100, vorzugsweise 40 - 60° C, in einem Lösungsmittelgemisch, das sowohl den Rhenium-oxo-Komplex als auch die Alkalimetallverbindung löst, umsetzt und daraus die das gewünschte Anion tragenden Rhenium-oxo-Komplexe durch Einengen zur Kristallisation bringt oder aber jeweils mit einem Überschuß der zum gewünschten Anion korrespondierenden Brönsted-Säure bei 20 - 180, vorzugsweise 40 - 120° C, in Lösungsmittelgemischen behandelt, die sowohl den Rhenium-oxo-halogeno-Komplex als auch die Brönsted-Säure lösen, und daraus die das gewünschte Anion tragenden rhenium-oxo-Komplexe durch Einengen zur Kristallisation bringt.

Das in Anspruch 4 erwähnte Reagenz Wasser braucht nicht eigens zugegeben zu werden, sondern dringt während des Rhenium-Einbaus in ausreichender Menge aus der Luft bzw. aus den Chemikalien oder Gefäßmaterialien in die Reagenzien ein, so daß im Zuge der Reaktion Dihalogeno-Rhenium-Gruppen zu Oxo-rhenium-Gruppen hydrolysiert werden.

Die Reaktionszeiten für den Einbau des Rheniums in das jeweilige Ausgangsporphyrin nach den Methoden des Anspruchs 4 liegt etwa im Bereich von 1 bis 20 h, meist aber bei 2 h.

Der Endpunkt der Reaktion kann spektralphotometrisch festgestellt werden. Es erscheint das UV/Vis-Spektrum des gebildeten Rhenium-Porphyrins anstelle des UV-Vis-Spektrums des Ausgangsporphyrins. Einen groben Anhaltspunkt liefert bereits die dunkelgrüne Farbe der Lösung des Endprodukts.

Als zur Vereinheitlichung der Zusammensetzung des Anions geeignete Bronsted-Säuren kommen alle Verbindungen in Frage, welche die in Anspruch 2 genannten Anionen bzw. aber auch $F^-$, $O/2^-$, $OCH_3^-$ und $C_6H_5O^-$ (bei Substituenten der Grundkörper gemäß Anspruch 3) in Kombination mit dem Proton enthalten.

Bei der nach beiden erfindungsgemäßen Herstellungsvarianten durchgeführten Säulenchromatographie werden übliche Adsorbentien, wie Aluminiumoxide oder Kieselgele, eingesetzt. Als Eluens für die Abtrennung des nicht umgesetzten Porphyrins wird ein schwach polares Lösungsmittel, wie Chloroform oder Toluol oder ein Gemisch aus beiden und für die Abtrennung der Rheniumkomplexfraktion (Hauptfraktion) ein Gemisch aus einem schwach polaren (z. B. Chloroform oder Dichlormethan) und einem stark polaren Lösungsmittel (Methanol, Ethanol, Propanol oder Aceton) eingesetzt. Besonders gut eignet sich ein Gemisch aus 50 Vol.% Chloroform und 50 Vol.% Methanol.

Mit den beschriebenen neuen Katalysatoren können die verschiedensten Olefine mit Wasserstoffperoxid epoxidiert werden. Dabei kann in organischen Lösungsmitteln, insbesondere solchen, welche ein Übertreten von Wasserstoffperoxid aus wäßriger Phase in die organische Phase gestatten, gearbeitet werden.

Die anzuwendenden Mengen an Katalysator können in einem weiten bereich liegen. Die im Einzelfall anzuwendende Katalysatorkonzentration kann, entsprechend dem Typ der vorgesehenen Rhenium-porphyrinverbindung sowie entsprechend der Reaktivität des jeweils umzusetzenden Olefins gewählt werden. Sie liegt in einem Konzentrationsbereich, der im allgemeinen 1/10000 bis 1/2 mol, vorzugsweise 1/5000 bis 1/5 mol pro mol Wasserstoffperoxid beträgt.

Die einmal eingesetzten Katalysatoren lassen sich nach geeigneter Abtrennung des Reaktionsgemisches für weitere Ansätze wiederverwenden.

Die Reaktionstemperaturen können in einem breiten Bereich liegen. Sie hängen ab von der jeweiligen Aktivität des verwendeten Katalysators, der Reaktivität des verwendeten Olefins, der Neigung des gewünschten Oxirans zur Ringöffnung und der Art des Lösungsmittels. Sie liegen im allgemeinen bei 0 bis 150, vorzugsweise 20 bis 120, insbesondere 20 bis 80 ° C. Die Reaktionszeiten liegen normalerweise bei 10 bis 24 Stunden. Die reaktionen können unter Atmosphärendruck oder bei höheren Drucken durchgeführt werden, solange das Reaktionssystem in flüssiger Phase gehalten werden kann.

Vorzugsweise wird in einem Druckbereich zwischen 1 und 50 bar gearbeitet.

Die mit der Erfindung erzielbaren Vorteile sind:

- Sehr kurze Reaktionszeiten
- Hohe Selektivität (kaum Nebenprodukt)
- Niedrige Katalysatorkonzentration
- Hohe chemische Stabilität des Katalysators, insbesondere gegenüber dem Epoxidationsmittel
- Keine oder nur minimale $H_2O_2$-Zersetzung
- Leichte Abtrennbarkeit und Wiederverwendbarkeit des Katalysators
- Vereinfachte Synthese der Katalysatoren

Die Erfindung wird im folgenden anhand von Ausführungsbeispielen zu den einzelnen Herstellungsmethoden gemäß Erfindung unter Charakterisierung der Verfahrensprodukte weiter erläutert.

Hinsichtlich des Nachweises der hervorragenden anwendungstechnischen Eigenschaften der erfindungsgemäß zugänglich gemachten Rhenium-oxo-Komplexe wird auf die gleichzeitig eingereichte Parallelanmeldung P 37 31 690.7-42 verwiesen.

Die folgenden Ausführungsbeispiele 1 - 13 geben komplette Darstellungsvorschriften für bestimmte Rhenium-oxo-Komplexe an. Die Beispiele 1 - 3 und 11 beschreiben die Kombination der Rhenium-Einführung und der Einführung eines definierten Anions in den Komplex. Die übrigen Beispiele zeigen den Austausch der Axial-Liganden am Oxorhenium(V)-Porphyrin.

Zur Charakterisierung der Verbindungen dienen Elementaranalysen, UV-VIS-, IR- und Massenspektren. Die Lage der Banden im sichtbaren Spektralbereich wird bei $Re^V$-Porphyrinen sehr stark vom Anion beeinflußt, das in trans-Stellung zur Oxogruppe steht.

Vorab wird eine Übersicht über die einzelnen Beispiele gegeben.

Für die Strukturformel und die in den Beispielen verwendete Bezifferung der neuen Rheniumkomplexe gilt:

ReO(TTP)X

| Nr. | X | Bemerkungen |
|-----|-----|-------------|
| 1 | O/2 | μ-Oxo-Komplex |
| 2 | OR | mit R gemäß a - e |
| 3 | F | |
| 4 | Cl | |
| 5 | Br | |
| 6 | I | |
| 7 | OCN | |
| 8 | NCS | |
| 9 | OClO₃ | Perchlorat |

| Nr. | R |
|-----|-----|
| a | Me |
| b | Et |
| c | iPr |
| d | tBu |
| e | Ac |

Beispiel 1 μ-Oxobis[oxo{meso-tetra(p-tolyl)porphyrinato}rhenium(V)], [ReO(TTP)]₂O (1)

1.1 Gewinnung aus metallfreiem Porphyrin

Eine Lösung von 1 g (1.5 mmol) $H_2(TTP)$ in 70 ml Trichlorbenzol (TCB) wurde mit 670 mg (2 mmol) $ReCl_5$ 2 h zum Rückfluß erhitzt. Der Verlauf der Reaktion wurde anhand des UV/VIS-Spektrums verfolgt. Nachdem sich nach ca. 2 h kein freies Porphyrin mehr im Spektrum nachweisen ließ, wurde das TCB im Vakuum entfernt und der Rückstand im Hochvakuum bei 60° C von den letzten Spuren des TCB befreit. Der Rückstand wurde an $Al_2O_3$ (Aktivität III, basisch, 20 x 5 cm) mit Toluol/$CHCl_3$ (4 : 1) chromatographiert. Der grüne, nicht weiter identifizierte Vorlauf wurde verworfen. Das bräunlich-grüne Hauptprodukt wurde mit MeOH/$CH_2Cl_2$ (1 : 1) eluiert und das Lösungsmittel im Vakuum entfernt. Hiernach wurde in 25 ml $CHCl_3$ unter Zugabe von 2 ml 2 n KOH gelöst und 12 h gerührt. Das organische Lösungsmittel wurde durch gelindes Erwärmen entfernt, der ausgefallene Komplex abfiltriert und mit destilliertem Wasser neutral gewaschen. Kristallisation aus Toluol/$CH_2Cl_2$ ergab 1,11 g 1
(83.9 %) als blaugrünes, schuppiges Pulver

$C_{96}H_{72}N_8O_3Re$ (MG) 1756.4)
Ber.: C 65.59 H 4.13 N 6.37
Gef.: C 65.71 H 4.22 N 6.29

UV/VIS ($\lambda_{max}$, log $\epsilon$): 345(4.94), 462(5.29), 599(4.19), 640(3.95) nm.

IR (KBr): 725 (Re-O), 670 cm$^{-1}$ (Re-O), typisch für $\mu$-Oxo-Brücke.

1.2 Darstellung von [ReO(TTP)]$_2$O (1) aus ReO(TTP)Cl (4)

Eine Lösung von 200 mg ReO(TTP)Cl (0.21 mmol) in 25 ml $CHCl_3$ wurde mit 10 ml 2 n KOH über Nacht gerührt. Hiernach erwärmte man schwach, um das Lösungsmittel zu verdampfen, filtrierte den ausgefallenen Komplex ab und wusch ihn mit destilliertem Wasser gründlich neutral. Nach Trocknen des Produkts im Vakuum ergab Kristallisation aus Toluol 141 mg (76.4 %) blaugrüne Kristalle, die spektroskopisch identisch mit dem in Versuch 1.1 erhaltenen Produkt waren.

Beispiel 2: Methoxo-oxo[meso-tetra(p-tolyl)porphyrinato]rhenium(V), ReO(TTP)OMe (2a)

2.1 Gewinnung aus metallfreiem Porphyrin

Eine Lösung von 1 g (1.5 mmol) $H_2(TTP)$ in 70 ml Trichlorbenzol (TCB) wurde mit 670 mg (2 mmol) $ReCl_5$ versetzt und 2 h unter Rückfluß erhitzt. Die Reaktion wurde UV/VIS-spektroskopisch kontrolliert. Nach 2 h war kein freies Porphyrin mehr nachzuweisen. Das TCB wurde im Vakuum entfernt und der Rückstand im Hochvakuum bei 60° C von den letzten Spuren des TCB befreit und an $Al_2O_3$ (Aktivität III, neutral, 20 x 5 cm) mit Toluol/$CHCl_3$ (4 : 1) chromatographiert. Der grüne, nicht weiter untersuchte Vorlauf wurde verworfen und die bräunlich-grüne Hauptfraktion mit MeOH/$CHCl_3$ (1 : 1) eluiert. Nach dem Entfernen des Lösungsmittels im Vakuum wurde die Lösung des Rückstandes in $CH_2Cl_2$ mit 100 ml MeOH versetzt und 4 h unter Rückfluß erhitzt. Das Lösungsmittel wurde im Vakuum abgezogen und der Rückstand aus MeOH/$CH_2Cl_2$ kristallisiert. Man erhielt 1,28 g 2a (94.1 %) in Form eines grünen kristallinen Pulvers.

$C_{49}H_{39}N_4O_2Re$ (MG 902.1)

Ber.: C 65.23 H 4.36 N 6.21
Gef.: C 65.07 H 4.11 N 6.08

UV/VIS ($\lambda_{max}$, log $\epsilon$): 346(4.49, 462(4.96), 588(4.04), 656(3.86), 674(3.74) nm.

IR (KBr): 2790 (OCH$_3$), 932 (RE = O), 725 CM$^{-1}$ (RE-O) MS: 902 (M +)

6

## 2.2 Gewinnung aus ReO(TTP)Cl (4)

Zu einer Lösung von 200 mg (0.22 mmol) ReO(TTP)Cl in 30 ml $CHCl_3$ wurden 20 ml MeOH gegeben und unter Rückfluß erhitzt. Die anfänglich tiefrote Lösung begann sich grün zu färben. Mit UV/VIS-spektroskopischer Analyse ließ sich nach 4 h kein freies Porphyrin im Spektrum mehr nachweisen. Das Lösungsmittel wurde im Vakuum entfernt und aus MeOH kristallisiert. Man erhielt 165 mg (81.8 %) dunkelgrüner Kristalle, die nach dem Trocknen im Vakuum die gleichen spektroskopischen Eigenschaften wie die in Versuch 2.1 erhaltenen Kristalle aufwiesen.

## Beispiel 3: Chloro-oxo[meso-tetra(p-tolyl)porphyrinato]rhenium(V), ReO(TTP)Cl (4)

### 3.1 Gewinnung aus metallfreiem Porphyrin

Eine Lösung von 1 g (1.5 mmol) $H_2$(TTP) in 70 ml Trichlorbenzol (TCB) wurde mit 670 mg (2 mmol) $ReCl_5$ versetzt und 2 h unter Rückfluß erhitzt. Danach war im UV/VIS-Spektrum kein freies Porphyrin mehr nachweisbar. Das Lösungsmittel wurde im Vakuum entfernt und der Rückstand 1 h im Hochvakuum bei 60° C von noch anhaftendem TCB befreit. Das Produkt wurde an $Al_2O_3$ (Aktivität III, neutral, 20 x 5 cm) mit $CHCl_3$/Toluol (4 : 1) chromatographiert. Der bräunliche Vorlauf wurde nicht weiter untersucht und verworfen. Die bräunlich-grüne Hauptfraktion wurde mit MeOH/$CHCl_3$ (1 : 1) eluiert und das Lösungsmittel im Vakuum abgezogen. In die Lösung des Rückstandes in 250 ml Toluol wurde vorsichtig mit konzentrierter Schwefelsäure vorgetrocknetes Salzsäuregas eingeleitet. Nach 10 min Rückfluß-Sieden am Wasserabscheider begann sich die anfänglich schwarzgrüne Lösung tiefrot zu färben. Ungefähr 2 h später änderte sich das UV/VIS-Spektrum nicht mehr. Nach Entfernen des Lösungsmittels im Vakuum ließen sich durch Kristallisation aus Toluol 1.19 g 4 (88.1 %) in Form von rotschwarzen Kristallen erhalten.

$C_{48}H_{36}N_4OReCl$ (MG 906.6)

Ber.: C 63.54 H 3.97 N 6.17
Gef.: C 63.40 H 4.09 N 5.85

UV/VIS ($\lambda_{max}$, log $\epsilon$): 338(4.70), 524(4.51), 654(4.02), 674(3.95) nm

IR (KBr): 970 (Re = O), 725 $cm^{-1}$ (Re-O)
MS: 906 ($M^+$)

### 3.2 Gewinnung aus [ReO(TTP)]$_2$O (1)

Eine Lösung von 140 mg [ReO(TTP)]$_2$) (0.08 mmol) in 20 ml $CHCl_3$ wurde mit 2 ml konzentrierter Salzsäure (37 Gew.%ige wäßrige Lösung) über Nacht gerührt, das Lösungsmittel verdampft und der Rückstand mit destilliertem Wasser neutral gewaschen. Kristallisation aus Toluol bei -15° C ergab 117 mg 4 (81.3 %) rotschwarze Täfelchen, die spektroskopisch identisch mit dem in Versuch 3.1 erhaltenen Material waren.

## Beispiel 4: Fluoro-oxo[meso-tetra(p-tolyl)porphyrinato]rhenium(V), ReO(TTP)F (3)

### 4.1 Gewinnung aus dem Methoxid 2a

Eine Lösung von 150 mg (0.17 mmol) ReO(TTP)OMe in 15 ml $CH_2Cl_2$ wurde mit 2 ml HF (40 % wäßrige Lösung) versetzt. Man ließ unter Rühren eindunsten und wusch den Rückstand mit destilliertem Wasser neutral. Kristallisation aus Toluol/$CHCl_3$ ergab 120 mg 3 (78.9 %) dunkelgrüne Schüppchen.

$C_{48}H_{36}N_4OReF$ (MG 890.1)

Ber.: C 64.71 H 4.04 N 6.29
Gef.: C 61.92 H 3.79 N 5.99 Versuch 4.1
C 62.02 H 3.91 N 6.03 Versuch 4.2

UV/VIS ($\lambda_{max,}$ log $\epsilon$): 349(4.62), 476(4.93), 606(3.98), 652(3,84) nm

IR (KBr): 972 (Re = O), 725(Re-O) 675 cm$^{-1}$ (Re-F)
MS: 890 (M$^+$)

4.2 Gewinnung aus [ReO(TTO)]$_2$O (1)

Eine Lösung von 140 mg (0.08 mmol) [ReO(TTP)]$_2$O in 20 ml CHCl$_3$ wurde mit 2 ml HF (40 Gew.%ige wäßrige Lösung) versetzt und 24 h gerührt. Der durch Erwärmen vom Lösungsmittel befreite, gründlich mit destilliertem Wasser neutral gewaschene Komplex wurde aus Toluol kristallisiert. Man erhielt 120 mg (84.4 %) dunkelgrüne Schüppchen eines spektroskopisch mit 3 identischen Materials.

Beispiel 5: Bromo-oxo[meso-tetra(p-tolyl)porphyrinato]rhenium(V), ReO(TTP)Br (5)

5.1 Gewinnung aus dem Methoxid 2a

Zu einer Lösung von 200 mg (0.22 mmol) ReO(TTP)OMe in 20 ml CH$_2$Cl$_2$ wurden 2 ml HBr (40 Gew.%ige wäßrige Lösung) getropft. Man ließ unter vorsichtigem Erwärmen eindunsten und wusch den abfiltrierten Rückstand mit destilliertem Wasser neutral.
Kristallisation aus Toluol/CHCl$_3$ ergab 185 mg 5 (83.5 %) schwarzes Kristallpulver.

C$_{48}$H$_{36}$N$_4$OReBr (MG 951)

Ber.: C 60.56 H 3.78 N 5.89
Gef.: C 60.68 H 3.79 N 5.72

UV/VIS ($\lambda_{max,}$ log $\epsilon$): 346(4.83), 532(4.50), 666(3.75), 710(3.76) nm

IR (KBr): 975 (RE = O), 727 cm$^{-1}$ (Re-O)
MS: 871 (ReO/TTP$^+$)

5.2 Gewinnung aus [ReO(TTP)]$_2$O (1)

Eine Lösung von 140 mg (0.08 mmol) [ReO(TTP)]$_2$O in 25 ml CHCL$_3$ wurde mit 2 ml HBr (40 Gew.%ige wäßrige Lösung) versetzt und unter schwachem Erwärmen gerührt. Nachdem alles Lösungsmittel verdampft war, wurde der Rückstand gründlich mit destilliertem Wasser neutral gewaschen und aus Toluol kristallisiert. Man erhielt 132 mg 5 (86.8 %) als schwarzes Kristallpulver, das spektroskopisch identisch mit dem aus Versuch 5.1 erhaltenen Pulver war.

Beispiel 6: Iodo-oxo[meso-tetra(p-tolyl)porphyrinato]rhenium(V), ReO(TTP)I (6)

6.1 Herstellung aus dem Methoxid 2a

Eine Lösung von 100 mg ReO(TTP)OMe in 10 ml CHCl$_3$ wurde mit 1 ml HI (57 Gew.%ige Lösung in Wasser) versetzt.
Man ließ eindunsten und wusch den Komplex mit distilliertem Wasser neutral. Um Spuren von freiem Iod zu beseitigen, wurde der Komplex 1 Tag bei 60° C im Hochvakuum ausgeheizt. Durch Kristallisation aus Toluol/CHCl$_3$ erhielt man 72 mg 6 (65.1 %) in Form dunkelgrün schimmernder Kristalle.

$C_{48}H_{36}N_4ORel$ (MG 998)

Ber.: C 57.71 H 3.61 N 5.61
Gef.: C 54.08 H 3.42 N 5.29

UV/VIS ($\lambda_{max}$, log $\epsilon$): 356(3.56), 492(4.42), 654(3.53) nm

IR (KBr): 967 (Re = O), 727 cm$^{-1}$ (Re-O)
MS: Keine Ionen

6.2 Herstellung aus [ReO(TTP)]$_2$O (1)

Eine Lösung von 140 mg [ReO(TTP)]$_2$O (0.08 mmol) in 25 ml CHCl$_3$ wurde mit 2 ml HI (57 Gew.%ige wäßrige Lösung) versetzt und das Lösungsmittel durch vorsichtiges Erwärmen entfernt. Man wusch den ausgefallenen Komplex mit destilliertem Wasser neutral und beseitigte Spuren von freiem Iod im Vakuum bei 50°C. Kristallisation aus Toluol ergab 112 mg 6 (69.9 %) dunkelgrüne Kristallplättchen, die spektroskopisch identisch mit dem aus Versuch 6.1 erhaltenen Produkt waren.

Beispiel 7: Cyanato-oxo-[meso-tetra(p-tolyl)porphyrinato]rhenium(V), ReO(TTP)OCN (7)

Eine Lösung von 200 mg ReO(TTP)Cl (0.22 mmol) in 20 ml CHCl$_3$ wurde mit etwa 100 mg KOCN und 5 Tropfen Eisessig versetzt. Man ließ unter Rühren eindunsten, wusch den abfiltrierten Komplex gründlich mit destilliertem Wasser neutral und trocknete 2 h im Hochvakuum bei 60° C. Kristallisation aus Toluol/CHCl$_3$ ergab 182 mg 7 (90.5 %) als tiefgrünes, kristallines Pulver.

$C_{49}H_{36}N_5O_2Re$ (MG 913.1)

Ber.: C 64.39 H 3.94 N 7.67
Gef.: C 63.04 H 4.20 N 7.57

UV/VIS ($\lambda_{max}$, log $\epsilon$): 340(4.61), 380(4.66), 512(4.46), 618(3.67) nm

IR (KBr): 2192 (N = C = O), 970 (Re = O), 727 cm$^{-1}$ (Re-O)
MS: 913 (M$^+$)

Beispiel 8: Thiocyanato-oxo[meso-tetra(p-tolyl)porphyrinato]rhenium(V), ReO(TTP)NCS (8)

Eine Lösung von 150 mg ReO(TTP)Cl (0.16 mmol) in 20 ml CHCl$_3$ wurde mit einer Spatelspitze KSCN und 5 Tropfen Eisessig versetzt. Man ließ über Nacht bei gelindem Erwärmen rühren und wusch den ausgefallenen Komplex mit destilliertem Wasser neutral. Anschließend wurde der Komplex im Vakuum 1 Tag getrocknet.
Kristallisation aus Toluol/CHCl$_3$ ergab 135 mg 8 (90.8 %) als schwarzgrünes Kristallpulver.

$C_{49}H_{36}N_5OReS$ (MG 929.9) Ber.: C 63.28 H 3.87 N 7.53
Gef.: C 60.17 H 3.87 N 7.25

UV/VIS ($\lambda_{max}$, log $\epsilon$): 334(4.71), 370(4.65), 520(4.54), 646(3.43) nm

IR (KBr): 2015 (N = C = S), 972 (Re = O), 727 cm$^{-1}$ (Re-O)
MS: 929 (M$^+$).

Beispiel 9: Perchlorato-oxo[meso-tetra(p-totyl)porphyrinato]rhenium(V), ReO(TTP)OClO$_3$ (9)

Eine Lösung von 120 mg [ReO(TTP)]$_2$O (0.06 mmol) in 10 ml CHCl$_3$ wurde mit 2 ml Perchlorsäure (7 Gew.%ige wäßrige Lösung) versetzt. Man erwärmte leicht, filtrierte den ausgefallenen Komplex ab und wusch mit destilliertem Wasser neutral. Nach Trocknen im Vakuum bei 50° C ergab Kristallisation aus Toluol 79 mg 9 (76.5 %) in Form von schwarzgrünem Kristallpulver.

C$_{49}$H$_{36}$N$_4$O$_5$ReCl (MG 970) Ber.: C 59.38 H 3.71 N 5.77
Gef.: C 56.40 H 3.52 N 5.59

UV/VIS ($\lambda_{max,}$ log $\epsilon$): 326(4.65), 358(4.62), 500(4.59), 624(3.70) nm

IR (KBr): 990 (Re = O), 727 cm$^{-1}$ (Re-O)
MS: 970 (M$^+$)

Beispiel 10: Ethoxo-oxo[meso-tetra(p-tolyl)porphyrinato]rhenium(V), ReO(TTP)OEt (2b)

Eine Lösung von 200 mg [0.22 mmol] ReO(TTP)Cl in 10 ml CHCl$_3$ wurde mit ca. 50 mg NaOEt versetzt und mit 20 ml EtOH 4 h unter Rückfluß erhitzt. Das Lösungsmittel wurde im Vakuum entfernt, und der Komplex wurde an Al$_2$O$_3$ (Aktivität III, neutral, 10 x 3 cm) chromatographiert. Als Laufmittel wurde Toluol/CHCl$_3$ (4 : 1) verwendet, als Eluens für das grünbraune Hauptprodukt diente EtOH/CHCl$_3$ (1 : 1). Nachdem das Lösungsmittel im Vakuum entfernt worden war, wurde in EtOH gelöst, mit 5 Tropfen HOAc versetzt und 4 h unter Rückfluß erhitzt. Das EtOH wurde im Vakuum abgezogen und aus EtOH kristallisiert. Man erhielt 152 mg 2b (75 %) als grüne Kristalle.

C$_{50}$H$_{41}$N$_4$O$_2$Re (MG 916.1) Ber.: C 65.50 H 4.47 N 6.11
Gef.: C 65.26 H 4.36 N 5.98

UV/VIS ($\lambda_{max,}$ log $\epsilon$): 336(4.41), 466(4.25), 504(4.19), 656(3.70) nm

IR (KBr): 950 (Re = O), 727 cm$^{-1}$ (Re-O)
MS: 916 (M$^+$)

Beispiel 11: Isopropyl-oxo[meso-tetra(p-totyl)porphyrinato]rhenium(V), ReO(TTP)OiPr (2c)

Eine Lösung von 335 mg (0.5 mmol) H$_2$(TTP) in 50ml Trichlorbenzol (TCB) wurde mit 280 mg (0.75 mmol) ReCl$_5$ unter Rückfluß erhitzt. Im UV/VIS-Spektrum ließ sich nach ca.2 kein freies Porphyrin mehr nachweisen. Nach Entfernen des Lösungsmittels im Vakuum wurde der Rückstand im Hochvakuum 1 h von den letzten Resten TCB befreit und an Al$_2$O$_3$ (Aktivität II, sauer, 10 x 3 cm) mit Toluol/CHCl$_3$ (4 : 1) chromatographiert. Der bräunlich grüne Vorlauf wurde verworfen, das grüne. Hauptprodukt mit Isopropanol/CHCl$_3$ (1 : 1) eluiert und das Lösungsmittel im Vakuum abgezogen. Die Lösung des Rückstandes in Isopropanol wurde mit 5 tropfen Eisessig versetzt und 4 h unter Rückfluß erhitzt. Nach Abziehen des Lösungsmittels im Vakuum wurde der Komplex mit destilietem Wasser neutral gewaschen und aus Isopropanol kristallisiert. Man erhielt 297 mg 2c (64.1 %) als grüne Schuppen.

C$_{51}$H$_{43}$N$_4$O$_2$Re (MG 930.1) Ber.: C 65.81 H 4.62 N 6.02
Gef.: C 65.39 H 4.48 N 6.07

UV/VIS ($\lambda_{max,}$ log $\epsilon$): 346(4.45), 464(4.73), 586(3.63) nm

IR (KBr): 935 (Re = O), 727 cm$^{-1}$ (Re-O)
MS: 930 (M$^+$)

Beispiel 12: tert-Butyloxo-oxo[meso-tetra(p-tolyl)porphyrinato]rhenium(V), ReO(TTP)OtBu (2d)

Eine Lösung von 135 mg (0.15 mmol) ReO(TTP)Cl in 15 ml CHCl$_3$ wurde mit 5 Tropfen Eisessig sowie 100 ml tert-Butanol 4 h zum Rückfluß erhitzt. Das Lösungsmittel wurde im Vakuum entfernt und der Rückstand mit destilliertem Wasser neutral gewaschen. Kristallisation aus tert-Butanol ergab 122 mg 2 d (86.7 %) in Form tiefgrüner Kristallplättchen.

C$_{52}$H$_{45}$N$_4$O$_2$Re (MG 944.1) Ber.: C 66.09 H 4.77 N 5.93
Gef.: C 65.61 H 4.66 N 5.85

UV/VIS ($\lambda_{max}$, log $\epsilon$): 344(4.66), 458(4.89), 584(4.03), 654(3.95), 674(3.85) nm

IR (KBr): 925 (Re = O), 670 cm$^{-1}$ (Re-O)
MS: 944 (M$^+$)

Beispiel 13: Acetato-oxo[meso-tetra(p-tolyl)porphyrinato]rhenium(V), ReO(TTP)OAc (2e)

Eine Lösung von 150 mg [ReO(TTP)]$_2$O (0.08 mmol) in 20 ml CHCl$_3$ wurde mit 1 ml Eisessig und 50 mg NaOAc versetzt und unter vorsichtigem Erwärmen vom Lösungsmittel befreit. Der abfiltrierte Komplex wurde mit destilliertem Wasser neutral gewachen und im Vakuum getrocknet. Kristallisation aus Toluol ergab 113 mg 2e (71.2 %) als grünes Kristallpulver.
C$_{50}$H$_{39}$N$_4$O$_3$Re (MG 929.8) Ber.: C 64.53 H 4.19 N 6.02
Gef.: C 64.78 H 4.29 N 5.92

UV/VIS ($\lambda_{max}$, log $\epsilon$): 348(4.60), 418(4.29), 464(4.75), 602(3.83), 654(3.69) nm

IR (KBr): 1670 (OAc, einzähnig), 967 (Re = O), 727 cm$^{-1}$ (Re-O)
MS: 930 (M$^+$)

## Ansprüche

1. Rhenium-oxo-Komplexe mit

- Octaethylporphyrin oder
- 5,10,15,20-Tetraphenylporphyrinen oder
- 5,10,15,20-Tetra(4-pyridyl)-porphyrinen als Liganden,

welche gegebenenfalls am Zentralatom ein Anion, außer O/2$^{2-}$($\mu$-oxo), CH$_3$O$^-$, C$_6$H$_5$O$^-$ oder F$^-$ bei den Grundkörpern, tragen.
2. Komplexe nach Anspruch 1,
**dadurch gekennzeichnet,**
daß das Anion Cl$^-$, Br$^-$, I$^-$, C$_2$H$_5$O$^-$, C$_3$H$_7$O$^-$, t-C$_4$H$_9$O$^-$, AcO$^-$, SCN$^-$, OCN$^-$ und ClO$_4$$^-$ ist.
    Komplexe nach Anspruch 1,
**dadurch gekennzeichnet,**
daß jeweils Wasserstoffatome oder freie elektronenpaare an den Phenyl- bw. Pyridylgruppen der Liganden ein-oder mehrmals substituiert sind durch Halogen, Hydroxy, Carboxy, Cyano, Rhodano, Nitro, C$_1$-C$_6$-Alkyl, Trihalogenmethyl, C$_1$-C$_6$-Alkoxy, C$_1$-C$_6$-Alkansulfonyloxy, Aminocarbonyl, einen oder zwei C$_1$-C$_6$-Alkylreste enthaltendes Aminocarbonyl, C$_1$-C$_6$-Alkylcarbonyl, Amino, Di-C$_1$-C$_6$-Alkylamino, C$_1$-C$_6$-Alkanoylamino, C$_1$-C$_6$-Alkyl-C$_1$-C$_6$-alkanoylamino, C$_1$-C$_6$-Alkansulfonylamino, C$_1$-C$_6$-Alkyl-C$_1$-C$_6$-alkansulfonylamino, Aminosulfonyl, einen oder zwei C$_1$-C$_6$-Alkylreste enthaltendes Aminosulfonyl, C$_1$-C$_6$-Alkoxysulfonyl (-SO$_2$-O-C$_1$-C$_6$-Alkyl), Sulfo oder C$_1$-C$_6$-Alkansulfonyl und zwei dieser Reste auch die Methylendioxygruppe sein können und das Anion gegebenenfalls Cl$^-$, Br$^-$, I$^-$, C$_2$H$_5$O$^-$, C$_3$H$_7$O$^-$, t-C$_4$H$_9$O$^-$, AcO$^-$, SCN$^-$, OCN$^-$ und ClO$_4$$^-$, aber auch F$^-$ und O/2$^{2-}$($\mu$-oxo),CH$_3$O$^-$ und C$_6$H$_5$O$^-$ ist.
4. Verfahren zur Herstellung von Rhenium-oxo-Komplexen, insbesondere derjenigen gemäß Ansprüchen 1 bsi 3,
**dadurch gekennzeichnet,**

EP 0 308 790 A2

daß man ein Mol Octaethylporphyrin, 5,10,15,20-Tetraphenylporphyrin oder 5,10,15,20-Tetra(4-pyridyl)-porphyrin mit mindestens einem Mol Rheniumpentahalogenid und 1 Mol Wasser in einem hochsiedenden, alle Reaktanden lösenden Solvens bei Temperaturen von 160 - 250, vorzugsweise 180 - 220° C, umsetzt, das Solvens vorzugsweise im Vakuum entfernt, den Rückstand nach Aufnehmen in einem organischen Lösungsmittel durch Säulenchromatographie fraktioniert, dann die in der Hauptfraktion vorliegenden, hinsichtlich der Zusammensetzung des Anions noch inhomogenen Komplexe entweder jeweils mit einer äquimolaren Menge einer gegebenenfalls in situ erzeugten Alkalimetallverbindung, welche das gewünschte Anion in Bindung mit dem Alkalimetall enthält, bei 0 - 100, vorzugsweise 40 - 60° C, in einem Lösungsmittelgemisch, das sowohl den Rhenium-oxo-Komplex als auch die Alkalimetallverbindung löst, umsetzt und daraus die das gewünschte Anion tragenden Rhenium-oxo-Komplexe durch Einengen zur Kristallisation bringt oder aber jeweils mit einem Überschuß der zum gewünschten Anion korrespondierenden Brönsted-Säure bei 20 - 180, vorzugsweise 40 -120° C, in Lösungsmittelgemischen behandelt, die sowohl den Rhenium-oxo-halogeno-Komplex als auch die Brönstedsäure lösen, und daraus die das gewünschte Anion tragenden Rhenium-oxo-Komplexe durch Einengen zur Kristallisation bringt.

12